# EUROPEAN PATENT SPECIFICATION

(11) **EP 2 544 717 B1**
(45) Date of publication and mention of the grant of the patent: **15.08.2018**
(21) Application number: 11709558.8
(22) Date of filing: 08.03.2011
(51) Int. Cl.: A61K 39/145

(54) **METHOD FOR PROTECTING AGAINST DISEASE CAUSED BY SECONDARY PATHOGENS**
VERFAHREN ZUM SCHUTZ GEGEN DURCH SEKUNDÄRE KRANKHEITSERREGER VERURSACHTE KRANKHEITEN
PROCÉDÉ DE PROTECTION CONTRE UNE MALADIE PROVOQUÉE PAR DES AGENTS PATHOGÈNES SECONDAIRES

(30) Priority: 10.03.2010 US 312380 P
(43) Date of publication of application: 16.01.2013
(73) Proprietor: Intervet International B.V., 5831 AN Boxmeer (NL)
(72) Inventor: LAKSHMANAN, Nallakannu, P., Millsboro DE 19966 (US); DESHPANDE, Muralidhar, S., Elkhorn NE 68022 (US); JAYAPPA, Huchappa, Gowda, Elkhorn NE 68022 (US); WASMOEN, Terri, Lee, Elkhorn NE 68022 (US)
(74) Representative: Intervet International B.V.
(86) International application number: PCT/US2011/027565
(87) International publication number: WO 2011/112593

(56) References cited:
- WO-A1-2005/002618
- WO-A2-01/74386
- WO-A2-2007/047938
- WO-A2-2007/118206
- APVMA: "NOTICE OF REGISTRATION OF AGRICULTURAL AND VETERINARY. CHEMICAL PRODUCTS", Gazette , February 2008 (2008-02), page 31PP, XP002641588, Retrieved from the Internet: URL:http://www.apvma.gov.au/publications/g azette/2008/02/gazette_2008-02-05.pdf [retrieved on 2011-06-14]
- DESHPANDE MURALIDHAR S ET AL: "Evaluation of the efficacy of a canine influenza virus (H3N8) vaccine in dogs following experimental challenge", VETERINARY THERAPEUTICS, vol. 10, no. 3, 1 October 2009 (2009-10-01), pages 103-112, XP009149084, VETERINARY LEARNING SYSTEMS, TRENTON, NJ, US ISSN: 1528-3593
- CASTLEMAN WILLIAM L ET AL: "Pathologic findings in dogs infected with newly emerged canine H3N8 influenza virus", FASEB JOURNAL, vol. 20, no. 4, Part 1, March 2006 (2006-03), page A214, XP09081167, & EXPERIMENTAL BIOLOGY 2006 MEETING; SAN FRANCISCO, CA, USA; APRIL 01 05, 2006 ISSN: 0892-6638
- YOON KYOUNG-JIN ET AL: "Influenza virus infection in racing greyhounds", EMERGING INFECTIOUS DISEASES, vol. 11, no. 12, 1 December 2005 (2005-12-01), pages 1974-1976, XP008080869, EID, ATLANTA, GA, US ISSN: 1080-6040
- CHALKER VICTORIA J ET AL: "The association of Streptococcus equi subsp. zooepidemicus with canine infectious respiratory disease", VETERINARY MICROBIOLOGY, vol. 95, no. 1-2, 29 August 2003 (2003-08-29), pages 149-156, XP002301527, ELSEVIER BV, NL ISSN: 0378-1135, DOI: 10.1016/S0378-1135(03)00155-X cited in the application
- LARSON LAURIE J ET AL: "Efficacy of the canine influenza virus H3N8 vaccine to decrease severity of clinical disease after cochallenge with canine influenza virus and Streptococcus equi subsp. zooepidemicus.", CLINICAL AND VACCINE IMMUNOLOGY : CVI, vol. 18, no. 4, 23 February 2011 (2011-02-23), pages 559-564, XP9149344, ISSN: 1556-679X

## Description

### Field of the Invention

Administration of a vaccine against a primary pathogen was found to protect against disease caused by a secondary, or opportunistic, pathogen. For example, canines were protected from disease caused by secondary pathogens, such as *Streptococcus equi* subspecies *zooepidemicus* (S. *equi*), by immunizing against collateral pathogens.

### Background of the Invention

*S. equi* has been associated with canine infectious respiratory disease (CIRD), which has been recognized as a multifactoral infection that primarily affects kenneled dogs (Chalker et al., Veterinary Microbiology 95 (2003):149-156). It was found, however, that *S*. *equi* could be isolated from asymptomatic, healthy, non-kenneled dogs. *S*. *equi* contributes to the severity of CIRD only in combination with other pathogens.

Canine influenza (CIV) has been identified as a H3N8 virus highly homologous with equine influenza virus. Canine influenza has been found to be virulent in certain types of dogs. For example, it was found to be the cause of an outbreak that killed several racing Greyhounds in kennels in Jacksonville, Florida. Although 100 per cent of exposed dogs become infected, only 80 percent have been found to manifest clinical signs of infection. Moreover, while some dogs exposed to CIV develop an acute disease with clinical signs of pneumonia, most dogs experience a milder syndrome such as a cough that persists for 10 to 21 days.

WO01/74386 discloses experimentally-generated cold-adapted equine influenza viruses that can be administered to an animal to treat respiratory disease such as, respiratory disease but not limited to, nasal discharge which is believed to be due directly or indirectly to equine herpes virus (EHV) or other viruses.

APVMA: "Notice of registration of agricultural and veterinary. Chemical products" of February 2008, discloses a canine vaccine comprising inactivated *Bordetella bronchiseptica* antigen and inactivated canine parainfluenza virus.

Deshpande Muralldhar S et al: Veterinary Therapeutics, Veterinary learning systems, vol. 10, no. 3,1 (2009), p103-112 discloses Canine influenza virus (CIV) subtype H3N8 and evaluates the efficacy of an inactivated CIV vaccine in 6-8 week old beagle pups. It discloses that lung damage caused by CIV can lead to secondary infections.

Castleman et al: FASEB J. vol. 20, no. 4, Part 1, (2006), page A214 discloses pathological findings in dogs infected with Canine H3N8 influenza virus. It further discloses that in spontaneous canine influenza infections, secondary bacterial pneumonia was common.

Yoon et al: Emerging Infectious Diseases, vol. 11, no. 12, (2005), p 1974-1976 discloses a study in an outbreak of respiratory disease in racing greyhounds. It was found that this outbreak was due to H3N8 influenza virus. It did not rule out a possible contribution to this outbreak from *S*. *zooepidemicus.*

WO 2005/002618 discloses a vaccine composition for use against canine infectious respiratory disease comprising any one or more of (a) an agent capable of raising an immune response in a dog against S. *zooepidemicus*; (b) an agent capable of raising an immune response in a dog against *M*. *cvnos* ; and (c) an agent capable of raising an immune response in a dog against a *Chlamydophila.* The vaccine may further comprise an agent capable of raising an immune response in a dog against canine parainfluenza virus CPIV.

WO2007/118206 discloses an isolated canine influenza virus (CIV) and discloses that the CIV may be used in a combination vaccine.

WO2007/047938 discloses isolated influenza virus that is capable of infecting canids and causing respiratory disease in the canid and discloses that the influenza virus may be used in a combination vaccine.

We have found that the severity of both *S*. *equi* and of CIV are enhanced by co-infection with the other.

### Summary of the Invention

A method for protecting a canine against disease caused by secondary pathogens comprises administering a vaccine against a primary pathogen. The canine to be protected is immunized with a CIV vaccine, thereby protecting the canine against disease caused by a secondary pathogen *S*. *equi.* As a consequence, the canine is protected against both CIV and *S. equi.*

### Brief Description of the Figures

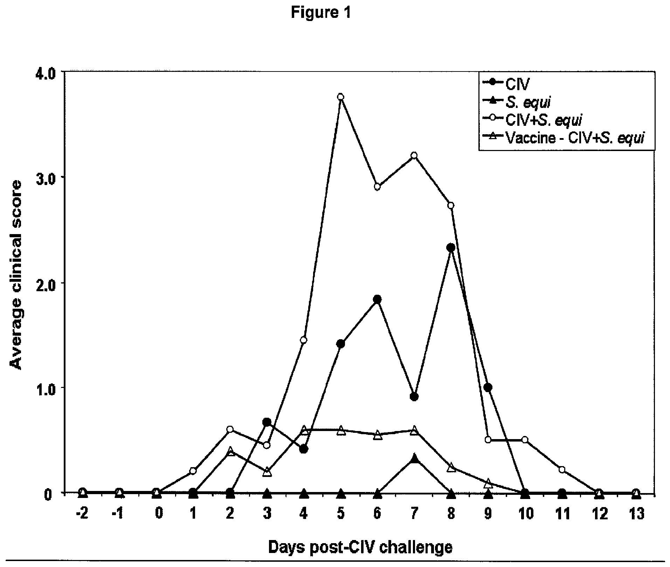

Figure 1, Clinical Scores following CIV challenge, charts the average clinical scores for each treatment group from two days before CIV challenge to 13 days post challenge. All dogs were challenged with CIV on study day 35 and monitored daily from day -2 through 13 days post-CIV challenge for clinical signs. Average clinical scores for each treatment group were plotted against days post-challenge. Refer to Table 4 for clinical scores.

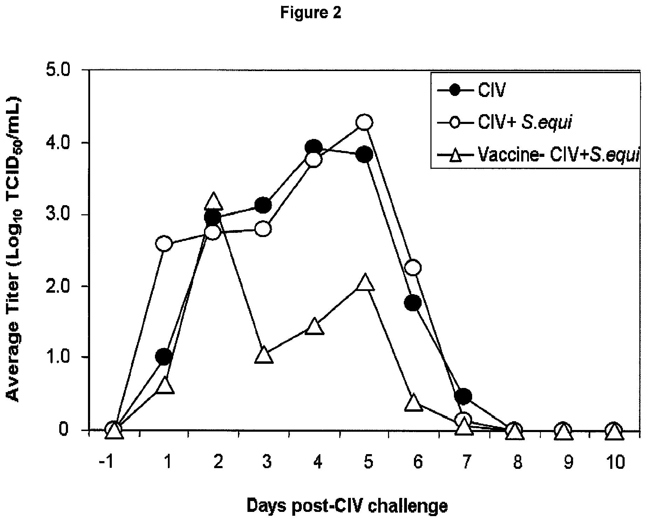

Figure 2, Nasal CIV shedding following challenge, charts the average virus titers for each group measured by collecting nasal swabs on each of days one through 10 post challenge. All dogs in Groups 1, 3 and 4 were challenged with CIV on day 35. Nasal swabs were collected on the day before challenge (day -1) to confirm that the pups were CIV free. Nasal virus shedding was monitored in challenged dogs by collecting nasal swabs daily for 10 days (day 1 through 10 post-challenge) and performing titration on MDCK monolayers. The average virus titers for each treatment group, expressed as Log₁₀ TCID₅₀/mL, were calculated and plotted against days post-challenge.

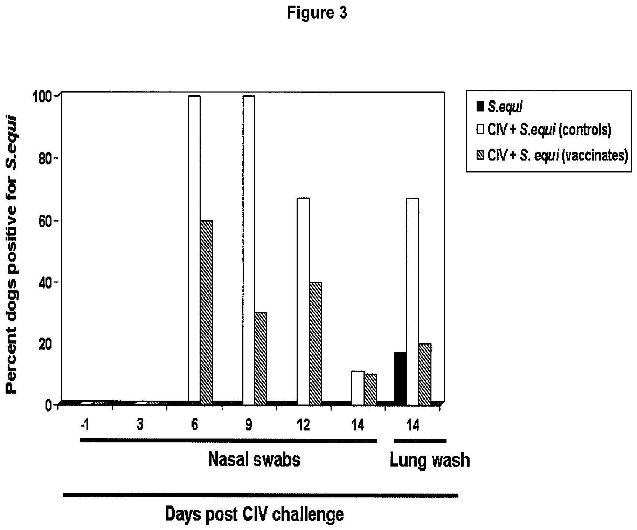

Figure 3, *S.equi* isolation from nasal swabs and lung lavage, charts the percentage of dogs in each group positive for *S*. *equi* from the day before CIV challenge to day 14 post challenge.

### Detailed Description of the Invention

The present invention is directed to a method for protecting canines against disease caused by secondary pathogens that do not present clinical signs in the absence of compromising factors, such as co-infection with another pathogen. In such cases, even though either pathogen by itself would not present clinical signs, the two together result in enhanced pathogenesis and clinically significant disease.

Canine influenza virus (CIV) subtype H3N8 may cause acute respiratory disease in certain dogs. We determined that *Streptococcus equi subsp. zooepidemicus* (*S. equi*) exacerbated the disease caused by CIV by investigating the pathogenesis following co-infection of dogs with CIV and *S*. *equi.* Similarly, we found that dogs infected with *S*. *equi* alone did not present clinical signs, but when challenged with both CIV and *S*. *equi,* active clinical disease was observed. We then found that immunization with a CIV vaccine protected dogs against dual challenge with virulent CIV and *S*. *equi.* Unexpectedly, respiratory disease caused by *S*. *equi* could be prevented by administering a vaccine against CIV.

Dogs challenged with *S*. *equi* alone did not exhibit any clinical signs, bacterial shedding or lung lesions. Dogs challenged with CIV alone developed some clinical signs, virus shedding and lung lesions. The clinical scores, CIV and/or *S.equi* shedding, and lung scores were substantially higher in dogs challenged with CIV plus *S.equi* when compared to in dogs challenged with CIV alone or *S.equi* alone. The lung scores, clinical scores and bacterial shedding were significantly lower in CIV vaccinated dogs challenged with CIV plus *S.equi* when compared to non-vaccinated dogs challenged with either CIV alone or CIV plus *S.equi.*

As used herein, the term primary pathogen refers to a pathogen that may cause clinical signs of a disease in some animals, but not necessarily of such severity that they can be detected in all animals exposed to the pathogen.

As used herein, the term secondary pathogen refers to a pathogen that is not likely to cause clinical signs of a disease in animals exposed to the pathogen unless the animal is weakened by exposure to another pathogen or is otherwise in a weakened state, also sometimes referred to as an opportunistic pathogen.

A pathogen that would by itself be a secondary pathogen and not likely cause clinical signs of disease may function as a primary pathogen when present with one or more other secondary pathogens.

CIV may be referred to as a primary pathogen and *S*. *equi* may be referred to as a secondary pathogen.

Kennel cough is an important disease in dogs caused by a number of viral and bacterial pathogens. CIV and *S.equi* are two new pathogens that have been isolated frequently in the recent years from canine respiratory disease cases, particularly in kennels and shelter dogs.

We investigated the pathogenesis of dual infection with CIV and *S.equi* in dogs and evaluated the efficacy of CIV vaccine in dual infection. Unlike the dogs infected with CIV alone, the majority of dogs infected with CIV plus *S.equi* exhibited clinical fever that lasted longer, and more severe clinical signs, particularly cough, that lasted longer. Nasal *S.equi* shedding in dual challenged dogs was very high with respect to the number of dogs positive for shedding and the duration of shedding. Furthermore, dual challenge induced substantially higher lung scores compared to either CIV alone or *S.equi* alone. We found dogs challenged with *S*. *equi* alone did not exhibit clinical fever, clinical signs of respiratory disease or lung lesions. Furthermore, *S.equi* was not detectable in the nasal secretions of *S.equi* alone-challenged dogs. We found that *S.equi* alone is not capable of inducing any disease and that *S.equi* is an opportunistic pathogen that in the presence of CIV leads to bacterial colonization. *S.equi* exacerbates the fever, other clinical signs and associated lung pathology caused by CIV. We discovered that administering CIV vaccine mitigated the clinical signs, the viral/bacterial shedding and the lung lesions induced, not only by CIV alone, but also CIV plus *S.equi.* The vaccination against CIV prevented respiratory disease caused by CIV and by opportunistic pathogens such as *S.equi.*

*S. equi* for challenge can be obtained from infected animals, such as the isolate used in the example that follows, or can be obtained from depositories, such as an isolate available from the ATCC, No. 6580.

Examples of canine influenza viruses that may be used for challenge, such as in the example below, and/or to prepare vaccines for administration according to the invention include any of the H3N8 viruses described in U. S. Patent Application No. 11/582,652, filed October 18, 2006 (Published Application US 2007/0092537), U. S. Patent Application No. 11/544,841, filed October 6, 2006 (Published Application US 2007/0082012), International Application No. PCT/US2006/04161, filed October 19, 2006, and U. S. Patent Application No. 11/956,658, filed December 14, 2007.

The CIV vaccines of the present invention will have avirulent, attenuated, or killed viruses, or comprise antigenic subunits. Avirulent and attenuated viruses are intended to include recombinant viruses, as well as avirulent isolates, passaged viruses, cold adapted viruses and viruses rendered less virulent or non-pathogenic by other means. The CIV vaccine may also be a commercial vaccine such as the CIV vaccine available from Intervet Inc./Schering-Plough Animal Health, Elkhorn, NE, NOBIVAC® Canine Flu H3N8. Any effective inactivated CIV vaccine may be used according to the invention.

The present invention further provides combination vaccines for eliciting protective immunity against canine influenza virus (CIV) and other diseases, e.g., other canine infectious diseases. Such vaccines will have avirulent, attenuated and/or killed viruses and/or bacteria, and/or comprise antigenic subunits. Accordingly, the present invention provides combination vaccines that include one or more strains of CIV, e.g., inactivated CIV H3N8, in combination with one or more other canine pathogens and/or immunogens, including, *e.g*., pathogens/immunogens for eliciting immunity to canine distemper virus, canine adenovirus, canine adenovirus type 2, canine parvovirus, canine parainfluenza virus, canine coronavirus, canine herpesvirus, canine pneumovirus, and/or *Leptospira* serovars, *e.g*., *Leptospira kirschneri* serovar grippotyphosa, *Leptospira interrogans* serovar canicola, *Leptospira interrogans* icterohaemorrhagiae, and/or *Leptospira interrogans* serovar pomona. Additional canine pathogens/immunogens that can be added to a combination vaccine of the present invention include *Bordetella bronchiseptica*; Leishmania organisms such as *Leishmania* major and *Leishmania infantum*; *Borrelia* species (spp.) spirochetes, including *B*. *burgdorferi* sensu stricto (ss), *B*. *burgdorferi* ss, *B. garinii*, and *B*. *afzelii*; a *Mycoplasma* species (*e.g*., *Mycoplasma cynos*); rabies virus; and *Ehrlichia canis.* In particular embodiments a combination vaccine of the present invention consists of CIV with canine parainfluenza virus (CPI) and/or canine adenovirus type 2 (CAV2) and/or *Bordetella bronchiseptica* (BB) and/or canine parvovirus (CPV), and/or canine distemper virus (CDV), and/or canine coronavirus, in any such combination. In one such embodiment the vaccine consists of canine influenza virus, *Bordetella bronchiseptica* and canine parainfluenza virus. In another such embodiment the vaccine consists of canine influenza virus, canine distemper virus, canine adenovirus type 2, canine parvovirus, and canine parainfluenza virus. In particular embodiments, when the combination vaccine of the present invention comprises one or more viral and/or bacterial strains, such strains are avirulent, attenuated, and/or killed.

The vaccines of the present invention may be administered by any route including: parenteral administration, intramuscular injection, subcutaneous injection, peritoneal injection, intradermal injection, oral administration, intranasal administration, scarification and combinations thereof. In a preferred embodiment of the invention, the vaccine is administered by intramuscular injection.

The vaccines of the present invention can either contain an adjuvant or not contain an adjuvant. Examples of adjuvants for use in the vaccines of the present invention include those containing an oil and water emulsion, and/or aluminum hydroxide. In the latter case, aluminum hydroxide from a commercial source can be used, for example, Alhydrogel, [Superfos Biosector, Frederikssund, Denmark] and Rehydrogel (Reheis Inc.). The oil and water emulsion typically comprises mineral oil or metabolizable oils (e.g., vegetable oil, fish oil). Non-ionic detergents or surfactants may be used as emulsifiers. Examples of emulsifiers include Tween 80/ Span 80, Arlecel 80/ Tween 80, and Montanides (Seppic, Paris, France). In the case of adjuvant emulsions, generally 5-25% of the volume is oil and 75-95% of the volume is aqueous. In some embodiments, the adjuvant emulsion is 20% oil and 80% aqueous by volume. The amount of aluminum hydroxide is usually between about 5% and 15% of the aqueous phase. In some embodiments, Emunade® is the adjuvant.

For some embodiments, ISCOM may be used as an adjuvant. ISCOM is an acronym for Immune Stimulating Complex and the technology was described by Morein et al. [Nature 308:457-460 (1984)]. An ISCOM can conveniently formed in one of two ways. In some embodiments, the antigen is physically incorporated in the structure during its formulation. In other embodiments, an ISCOM-matrix (as supplied by, for example, Isconova) does not contain antigen but is mixed with the antigen of choice by the end-user prior to immunization. After mixing, the antigens are present in solution with the ISCOM-matrix but are not physically incorporated into the structure.

The following example is provided for a more clear understanding of the invention. It is merely illustrative and is understood to not limit the scope or underlying principles of the invention in any way. Various modifications of the invention within the scope claimed will be apparent to those skilled in the art to which the invention pertains from the example and the description herein.

### Example

### Introduction

A total of 32 dogs, aged 7 to 8 weeks were used in the study. A group of 10 dogs was vaccinated with two doses of Canine influenza H3N8 killed virus vaccine, administered 21 days apart. The vaccinated dogs were challenged with virulent CIV and *S*. *equi,* two weeks after the booster vaccination. The remaining 3 groups of dogs were used to evaluate pathogenesis following single and dual challenge without vaccination. One group of 6 dogs was challenged with CIV alone and another group of 6 dogs was challenged with *S*. *equi* alone. A group of 10 dogs was challenged with both CIV and *S*. *equi.*

All dogs were monitored for clinical signs of respiratory disease and virus and/or bacterial shedding. Serum samples were collected prior to vaccination from the vaccinated group, before challenge administration from all groups, and at the time of necropsy from all groups to determine antibody titer to CIV. Dogs were euthanized at 14 days after the CIV challenge (Study day 49) and lungs were evaluated for lung lesions. At the time of necropsy, lung lavage samples were collected for *S.equi* isolation.

A commercial lot of Canine influenza H3N8 killed virus vaccine (Intervet Inc., Elkhorn, NE) was used to vaccinate 7-8 week old dogs in Group 4.

All dogs in Group 4 were CIV seronegative at first vaccination and all dogs in Groups 1, 2 and 3 were CIV seronegative at the time of CIV challenge.

**Table 1 Treatment Groups:**

| **Treatmen t groups** | **No. of pups** | **Vaccination Day** | **Challenge organisms and Challenge Day** | **Day of Necropsy** |
|---|---|---|---|---|
| 1 | 6 | NA | **Single** | Day 49 |
| | | | CIV - Day 35 | |
| 2 | 6 | NA | **Single** | Day 49 |
| | | | *S. equi-* Day 38 | |
| 3 | 10 | NA | **Dual** | Day 49 |
| | | | CIV- Day 35 | |
| | | | *S*. *equi* - Day 38 | |
| 4 | 10 | 0, 21 | **Dual** | Day 49 |
| | | | CIV- Day 35 | |
| | | | *S*. *equi* - Day 38 | |

Dogs were sorted by sex and litter and were assigned computer generated random numbers. The dogs were sorted by the random numbers in ascending order and assigned to treatment groups.

All 10 dogs in Group 4 were vaccinated with CIV vaccine by subcutaneous injection of 1 mL CIV vaccine (Serial # 219104) on study days 0 and 21.

Clinical Assessment: Clinical assessments were made and temperatures were recorded on study days 33 and 34 (two days prior to CIV challenge), and on the day of CIV challenge (study day 35) for all dogs. Clinical observations were performed following the Clinical Assessment Guide.

Sample Collection: Approximately 5 mL of blood samples were collected from all dogs on the day before CIV challenge administration (study day 34). Nasal swabs were also collected from all dogs in Groups 1, 3 and 4 on study day 34 to detect CIV shedding.

Canine Influenza Virus: The virus isolate CIV14-06A harvested at passage 3 (labeled as "Canine Influenza Virus, CIV14-06A (P3)) was used as challenge virus. This virus originally was isolated from a dog suffering with canine respiratory disease. On the day of challenge, frozen virus fluid was thawed immediately before use and diluted in sterile, cold Dulbecco's Modified Eagles Medium (DMEM) to a final volume of 2 mL per dog containing a targeted challenge dose of 7.5 Log₁₀TCID₅₀.

*Streptococcus equi subsp zooepidemicus:* The bacterial isolate was obtained from a dog residing in an animal shelter and died from canine respiratory disease complex. The bacteria were isolated, purified and characterized as *S.equi subsp zooepidemicus.* The challenge material was prepared by growing bacteria on Brain Heart Infusion (BHI) broth and diluting to obtain 1X10⁹ cfu/mL.

### Challenge Administration

Canine Influenza Virus: Dogs in Groups 1, 3 and 4 were challenged with CIV on day 35. For administering challenge, four dogs were placed in a Plexiglas chamber and 8 mL of challenge virus (2 mL/dog) was used to generate aerosol over a period of approximately 25-30 minutes. The dogs were exposed to the aerosol for a total of 40 minutes.

*S*. *equi subsp zooepidemicus:* Dogs in Groups 2, 3 and 4 were challenged with *S*. *equi* on study day 38. Half milliliter (0.5 mL) of challenge material containing 1x10⁹ cfu of *S*. *equi*/mL was instilled in each nostril of all dogs using a nasal cannula.

### Post-Challenge Monitoring

Clinical signs: Rectal temperature was recorded and clinical assessment was performed for each dog daily for 13 days post-challenge.

### Nasal Swab Collection

Canine Influenza Virus: Nasal swabs were collected from each dog daily for 10 days from the day of challenge in order to assess CIV shedding. Nasal swabs were collected in virus transport medium

*S.equi* isolation: Nasal swabs were collected for bacterial isolation using bacterial transport medium once in every three days following challenge until the day of necropsy.

Blood Collection: Blood samples (approximately 5-10 mL) were collected on the day of necropsy in order to evaluate antibody titers against CIV.

### Necropsy/Disposition

One dog (ID CAUELW) in Group 3 died on study day 43 (i.e., 8 days after challenge with CIV and 5 days after challenge with *S.equi*) due to severe respiratory distress. The dog exhibited fever, depression, severe coughing and dyspnea for two days prior to death. The necropsy revealed severe pneumonia characterized by fibrinous adhesions between lung lobes and chest wall, consolidation and micro-abscesses on lung lobes.

One dog (ID CAUELZ) in Group 1 exhibited fever, severe coughing, depression and dyspnea on study days 43 and 44. Therefore, the research veterinarian euthanized the dog on day 44 (9 days after CIV challenge) and performed necropsy. The lung lesions were characterized by consolidation suggestive of pneumonia.

Post-Challenge Necropsy Observations & Specimens: All remaining dogs were euthanized on day 14 post-CIV challenge. Dogs were euthanized immediately before necropsy to allow quick lung evaluation before post-mortem changes set in.. Lung washes from fresh lung tissues were also collected for bacterial isolation.

### CLINICAL ASSESSMENT GUIDE

Upon entering the isolation room, the personnel performing the clinical assessment walked through to assess the attitude of the animals and then continued with the clinical observations. Clinical observations were completed first for each dog before other procedures were conducted, such as challenge administration. The following guidelines were used for scoring each clinical sign.

### Nasal Discharge

0 = Absent
0.5 = Serous discharge: Thin, clear fluid dribbles from the nostril. Fluid must be running out of the nose to be recorded here.
1 = Mucopurulent discharge, mild to moderate: Cloudy fluid mixed with mucus runs at least halfway down from the nose to the mouth.
2= Mucopurulent discharge, severe: Mucus runs past the mouth.

### Ocular Discharge

0 = Absent: Small amount of dried crusted material in the corner of the eye is not considered an ocular discharge.
0.5 = Serous discharge: Clear fluid is discharged running down from eyes at least halfway down to the mouth. 1 = Mucopurulent discharge, mild to moderate: Cloudy fluid mixed with mucus runs at least halfway down from the eye to the mouth.
2 = Mucopurulent discharge, severe: Fluid or mucus runs halfway down the nose or rims the eye and soaks the hair at inner or outer corner of the eye.

### Cough

0 = Absent
0.5 = Mild: Only one brief cough is observed.
1.0 = Moderate: Cough is persistent, occurring repeatedly in the observation period.
2.0 = Severe: Cough is accompanied by choking or retching sounds.

| **Sneezing** | **Dyspnea** |
|---|---|
| 0 = Absent | 0 = Absent (Normal breathing) |
| 2 = Present | 2 = Present (Labored breathing) |

### Depression

0= Absent (Normal activity)
2= Present: Dog is less active or playful, compared to normal. Pups are recorded if lethargic or lying down and reluctant to stand while observation is conducted.

### Analytical Methods

Hemagglutination Inhibition (HAI) Assay: Antibodies to CIV in dog serum samples were measured by hemagglutination inhibition (HAI) assay. Briefly, a serial two-fold dilution of test serum was performed in PBS in U-bottomed 96-well microtiter plates. An equal volume (25 µL) of virus suspension containing 4 HA units of CIV25-06B was added to each well containing test serum, and the plates were incubated at 20-25°C for 60±5 minutes for antigen-antibody reaction to occur. Then, 50 µL of 0.5 % rooster RBC suspension was added. The plates were incubated at 20-25°C for 45-60 minutes and HAI results were read. The HAI titer was recorded as the reciprocal of the highest dilution of the serum showing hemagglutination inhibition. All assays were performed in duplicate and the endpoint HAI titer was determined.

CIV Titration in Cell Culture: To confirm the titer of challenge material and to measure virus shedding in challenge-administered dogs, the virus titers in challenge material, and nasal swab were determined by titration in MDCK cells. MDCK cells were seeded in 96-well tissue culture plates for 2-3 days, and were inoculated with ten-fold serially diluted virus suspension or samples prepared from nasal swabs. The plates were incubated at 36±2°C temperature and 4-6 % CO₂. Seven days post-infection, the plates were observed for cytopathic effect (CPE), and the 50 % end-point for infectivity was calculated using Spearman-Karber method. The virus titers were expressed as Log₁₀TCID₅₀/mL.

*S. equi* isolation: Sample supernatants were inoculated onto blood agar plates (10 µL) and the plates were incubated at 36±2°C for 24-48 hours. Colonies exhibiting hemolysis were further identified as *S.equi* by Gram's staining, morphology, colony characteristics and standard identification tests (such as oxidase, catalase and coagulase tests).

### Data Analysis

Outcome Variables: Lung lesion score was the primary variable. Other variables included clinical signs, virus shedding, bacterial shedding and serology.

### Statistical Analysis

Lung Lesion Scores: The percent consolidation of each lung lobe scored during necropsy was converted into weighted score based on the lung scoring system. Median lung scores for various groups were compared using Wilcoxon Exact Rank Sum tests. Mitigated fraction estimate of vaccine efficiency relative to non-vaccinated groups and the 95% confidence interval for the estimate is also reported.

Clinical Scores: Clinical scores for fever, ocular discharge, nasal discharge, sneezing, coughing, depression and dyspnea for 13 days post-challenge were summed to obtain a summed clinical score for each dog. The summed scores were compared between treatment groups using Wilcoxon Exact Rank Sum tests.

Virus Shedding: Virus titers (Log₁₀ TCID₅₀/mL) were summed over 10 days post-challenge for each dog. The mean area under curve (AUC) and number of days of virus shedding were analyzed using Wilcoxon Exact Rank Sum tests.

Bacterial Shedding: The number of days of bacterial shedding was analyzed using Wilcoxon Exact Rank Sum tests and the number of days positive for shedding was compared between treatment groups.

Seroconversion: Geometric mean antibody titers at different time points were reported.

Statistical analysis was performed using SAS version 9.1.3 (SAS Institute, Cary, NC, USA). Statistical significance was declared for p<0.05.

### Test Validity and Acceptability Criteria

All dogs, except those in Group 4, were negative for CIV antibodies at the time of CIV challenge.

All dogs, except those in Group 2, were negative for nasal CIV shedding at the time of CIV challenge. Dogs in Group 2 were not tested for CIV shedding.

All non-vaccinated dogs challenged with CIV (Groups 1 and 3) exhibited varying degrees of lung lesions following CIV challenge.

### RESULTS AND DISCUSSION

Validity: All dogs in Group 4 were CIV seronegative before the first vaccination, and all non-vaccinated dogs (Groups 1, 2 and 3) remained seronegative before CIV challenge (Table 2). All dogs were also negative for CIV and/or S. *equi* shedding at the time of CIV challenge (Tables 5 & 6).

Seroconversion: The HAI antibody titers were tabulated and compared between treatment groups (Table 2). All vaccinated dogs seroconverted to CIV following vaccination. The HAI antibody titers ranged between 10 and 160, with a GMT of 73 at day 34. The majority of the vaccinates (80%) developed an HAI titer of 40 or above. All dogs in non-vaccinated groups were CIV seronegative at the time of CIV challenge (HAI titer <10). Following challenge, antibody titers in vaccinated dogs reached very high levels (GMT>6378), demonstrating the efficacy of the vaccine in priming the immune system against virulent CIV. The HAI titers in all of these dogs were at least 2560. The non-vaccinated dogs challenged with CIV in Groups 1 and 3 seroconverted following CIV challenge with GMT of 226 and 278, respectively. All dogs in Group 2 remained CIV seronegative at the time of necropsy confirming that they were not exposed to CIV and biosecurity procedures were effective.

**Table 2: Hemagglutination inhibition antibody titer**

| **Treatment** | **Dog ID** | **Study Day** | | |
|---|---|---|---|---|
| | | **0** | **35** | **49** |
| CIV Challenge (Group 1) | CAUELZ | NT* | <10 | 80** |
| | CAUENF | NT | <10 | 320 |
| | CAUENR | NT | <10 | 640 |
| | CAUEMP | NT | <10 | 160 |
| | CAUELN | NT | <10 | 320 |
| | CAUEMZ | NT | <10 | 160 |
| | | **GMT** | **<10** | **226** |
| *S. equi* Challenge (Group 2) | CAUELS | NT | <10 | <10 |
| | CAUEME | NT | <10 | <10 |
| | CAUENM | NT | <10 | <10 |
| | CAUENU | NT | <10 | <10 |
| | CAUELM | NT | <10 | <10 |
| | CAUELR | NT | <10 | <10 |
| | | **GMT** | **<10** | **<10** |
| CIV plus *S. equi* Dual Challenge (Group 3) | CAUELU | NT | <10 | 320 |
| | CAUELW | NT | <10 | H*** |
| | CAUENK | NT | <10 | 320 |
| | CAUELX | NT | <10 | 960 |
| | CAUEPU | NT | <10 | 160 |
| | CAUENN | NT | <10 | 960 |
| | CAUEMR | NT | <10 | 160 |
| | CAUEMN | NT | <10 | 320 |
| | CAUELP | NT | <10 | 80 |
| | CAUENA | NT | <10 | 160 |
| | | **GMT** | **<10** | **278** |
| CIV Vaccination and CIV plus *S. equi* Dual Challenge (Group 4) | CAUELT | <10 | 160 | 5120 |
| | CAUEN W | <10 | 160 | 7680 |
| | CAUEPB | <10 | 160 | >10240^{A} |
| | CAUEMB | <10 | 40 | 5120 |
| | CAUENP | <10 | 160 | 2560 |
| | CAUEMS | <10 | 10 | >10240^{A} |
| | CAUENL | <10 | 120 | >10240^{A} |
| | CAUEML | <10 | 10 | >10240^{A} |
| | CAUELL | <10 | 160 | 5120 |
| | CAUEMX | <10 | 80 | 3840 |
| | **GMT** | **<10** | **73** | **6378** |

| | | | | |
|---|---|---|---|---|
| *NT= Not tested; **Sample collected at day 10 post-challenge; H***= Sample hemolyzed. Sample collected on day 9 post-challenge. ^{A} For samples with HI titer of >10240, the value of 10240 was used for the calculation of GMT | | | | |

Challenge Confirmation: Challenge virus samples frozen and stored at -50°C or colder before and after challenge administration were titrated on MDCK cells and the dose was confirmed to be 7.73 Log₁₀TCID₅₀ of CIV14-06A per dog. Additionally, there was no substantial difference between pre-challenge and post-challenge retention samples.

The *S.equi* retention sample titrated on blood agar plates confirmed the challenge material titer to be 1X10⁹ pfu/mL. The bacteria remained viable during the course of challenge administration.

Rectal Temperature and Clinical Signs: Rectal temperatures in all treatment groups were recorded daily from study day 33 through day 48 (Table 3). In Group 1 (CIV alone), two of the six dogs developed clinical fever (≥39.5°C) only for one day. One of the two dogs also developed severe depression and was euthanized. In Group 2, one of the six dogs (*S.equi* alone) developed mild fever only for one day. In Group 3 challenged with both CIV and *S*. *equi,* 90% (9 of 10) of the dogs developed fever and majority of them (70%) exhibited fever for at least two days. Fifty per cent of the vaccinated dogs (5 of 10, Group 4) also developed fever following dual challenge. However, the fever lasted only for one day.

**Table 3: Rectal Temperature**

| **Group** | **Dog ID** | **Study Day** | | | | | | | | | | | | | | | |
|---|---|---|---|---|---|---|---|---|---|---|---|---|---|---|---|---|---|
| | | **33** | **34** | **35** | **36** | **37** | **38** | **39** | **40** | **41** | **42** | **43** | **44** | **45** | **46** | **47** | **48** |
| CIV Challenge (Group 1) | CAUELZ | 38.8 | 38.5 | 38.7 | 38.9 | 38.8 | 38.6 | 38.4 | 39.2 | 38.5 | 38.4 | **40.4** | 35.6 | Euthanized on day 44 | | | |
| | CAUENF | 38.8 | 38.8 | 38.7 | 39.3 | 38.9 | 38.5 | 38.4 | 38.8 | 38.4 | 38.1 | 38.5 | 38.6 | 38.6 | 38.6 | 38.7 | 38.7 |
| | CAUENR | 38.9 | 38.6 | 39.1 | 39.2 | 39.1 | 39.1 | 38.9 | **39.5** | 39.2 | 38.9 | 38.9 | 38.6 | 39.1 | 38.6 | 38.6 | 38.7 |
| | CAUEMP | 39.0 | 38.5 | 38.5 | 39.4 | 38.6 | 38.4 | 38.5 | 38.8 | 38.7 | 38.5 | 38.6 | 39.0 | 39.0 | 38.8 | 38.8 | 38.9 |
| | CAUELN | 38.8 | 39.1 | 38.9 | 39.1 | 39.3 | 38.5 | 39.2 | 39.0 | 39.1 | 38.6 | 38.8 | 39.2 | 38.5 | 38.8 | 38.8 | 38.7 |
| | CAUEMZ | 38.8 | 38.7 | 38.9 | 38.6 | 38.7 | 38.8 | 38.7 | 39.0 | 38.6 | 38.9 | 38.7 | 38.9 | 38.6 | 38.5 | 38.8 | 38.7 |
| *S.equi* Challenge (Group 2) | CAUELS | 38.9 | 38.7 | 38.8 | 38.7 | 38.7 | 39.0 | 38.6 | 38.9 | 38.6 | 38.9 | 38.8 | 38.9 | 38.6 | 38.4 | 38.7 | 38.9 |
| | CAUEME | 38.9 | 38.8 | 38.4 | 38.5 | 39.0 | 39.0 | 38.7 | 38.8 | 38.4 | **39.6** | 39.1 | 38.7 | 38.3 | 38.6 | 38.7 | 38.8 |
| | CAUENM | 38.8 | 38.9 | 39.0 | 38.6 | 38.8 | 38.7 | 38.7 | 38.8 | 38.5 | 39.1 | 39.1 | 38.6 | 38.6 | 38.4 | 38.4 | 38.9 |
| | CAUENU | 38.9 | 38.9 | 38.5 | 38.4 | 38.3 | 38.6 | 38.9 | 38.6 | 38.4 | 38.9 | 38.8 | 38.6 | 38.7 | 38.6 | 38.5 | 39.1 |
| | CAUELM | 38.9 | 38.6 | 39.3 | 39.1 | 39.2 | 38.8 | 39.2 | 39.2 | 38.7 | 39.1 | 38.8 | 38.6 | 38.4 | 38.7 | 38.7 | 38.6 |
| | CAUELR | 38.9 | 38.6 | 38.8 | 38.7 | 38.7 | 38.6 | 38.9 | 39.1 | 38.5 | 38.8 | 38.9 | 38.9 | 38.6 | 38.4 | 38.6 | 38.9 |
| CIV plus *S.equi* Dual Challenge (Group 3) | CAUELU | 38.9 | 38.9 | 39.2 | 39.2 | **39.5** | 39.2 | **39.5** | 39.4 | **40.0** | 39.1 | 38.9 | 38.9 | 38.5 | 38.7 | 38.5 | 38.6 |
| | CAUELW | 38.9 | 38.7 | 38.5 | 39.3 | 38.8 | 39.1 | **39.6** | 39.2 | **39.7** | **39.7** | Died on day 43 | | | | | |
| | CAUENK | 38.9 | 38.5 | 38.7 | 38.8 | 38.6 | 38.7 | 39.1 | 39.1 | 39.2 | 38.8 | **39.9** | 38.7 | 38.4 | 38.8 | 38.7 | 38.7 |
| | CAUELX | 38.8 | 38.6 | 38.8 | 39.4 | 38.6 | 38.9 | 39.2 | **39.6** | 38.7 | **40.4** | 39.0 | 38.7 | 38.4 | 38.8 | 38.8 | 39.0 |
| | CAUEPU | 38.8 | 38.6 | 38.8 | 39.3 | 38.8 | 38.6 | 39.0 | 39.1 | 38.8 | 38.8 | 38.8 | 38.9 | 38.7 | 38.4 | 38.6 | 39.0 |
| | CAUENN | 38.8 | 38.9 | 38.9 | 39.2 | 39.2 | 38.9 | 38.7 | **39.5** | **40.4** | 39.1 | 39.4 | 38.8 | 39.0 | 38.8 | 38.8 | 38.6 |
| | CAUEMR | 38.8 | 38.6 | 39.1 | 39.1 | 38.9 | 38.7 | 39.4 | **39.5** | **39.8** | 39.1 | 39.1 | 39.1 | 38.7 | 38.9 | 38.8 | 38.7 |
| | CAUEMN | 38.9 | 38.5 | 38.8 | **40.1** | 38.7 | 39.2 | 39.4 | **40.1** | 38.9 | 38.5 | 38.8 | 39.1 | 38.5 | 38.5 | 38.6 | 38.6 |
| | CAUELP | 38.8 | 38.6 | 38.7 | 38.4 | 38.9 | 38.8 | **39.5** | **40.1** | 38.8 | 39.4 | 39.3 | 38.5 | 38.9 | 38.9 | 38.7 | 38.4 |
| | CAUENA | 38.8 | 38.6 | 39.0 | 38.9 | 39.2 | 39.3 | 39.1 | **39.7** | 39.4 | 39.0 | 39.0 | 38.5 | 38.5 | 38.9 | 38.5 | 38.7 |
| | CAUELT | 38.9 | 38.8 | 39.1 | 38.9 | **39.7** | 39.3 | 38.5 | 39.1 | 39.0 | 39.2 | 38.6 | 38.8 | 38.4 | 38.5 | 38.7 | 38.8 |
| | CAUENW | 38.9 | 38.3 | 39.2 | 38.6 | 39.1 | 39.1 | 38.3 | 39.0 | 38.6 | 38.8 | 38.8 | 39.1 | 38.3 | 38.6 | 38.7 | 38.7 |
| | CAUEPB | 38.8 | 38.5 | 38.7 | 38.7 | 39.2 | 38.8 | 38.3 | 38.6 | 38.5 | 39.2 | 38.7 | 38.5 | 38.4 | 38.8 | 38.5 | 38.7 |
| | CAUEMB | 38.8 | 38.7 | 39.2 | 38.5 | **39.5** | 38.7 | 38.4 | 38.8 | 38.8 | 38.8 | 38.9 | 38.4 | 38.7 | 38.6 | 38.7 | 38.8 |
| | CAUENP | 38.9 | 38.7 | 39.1 | 38.7 | 39.2 | 38.6 | 38.9 | 38.7 | 38.9 | 39.0 | 38.6 | 38.9 | 38.5 | 38.4 | 38.6 | 38.9 |
| | CAUEMS | 38.7 | 38.8 | 38.6 | 38.7 | 38.7 | 39.0 | 38.9 | 39.3 | 38.9 | **40.1** | 38.9 | 38.8 | 38.7 | 38.5 | 38.6 | 38.9 |
| | CAUENL | 38.8 | 39.3 | 38.7 | 38.8 | 39.4 | 39.1 | 38.7 | **39.6** | 38.8 | 39.4 | 39.1 | 38.7 | 38.6 | 38.6 | 38.7 | 39.0 |
| | CAUEML | 38.8 | 39.2 | 38.5 | 38.7 | 39.0 | 39.1 | 38.7 | 39.2 | 38.7 | 39.3 | 38.7 | 38.8 | 39.1 | 38.9 | 38.8 | 39.0 |
| | CAUELL | 38.7 | 38.3 | 38.7 | 38.9 | 38.9 | 39.2 | 38.5 | 39.4 | 38.7 | 38.9 | 39.1 | 38.6 | 39.0 | 38.9 | 38.7 | 38.7 |
| | CAUEMX | 38.8 | 38.7 | 38.8 | 38.7 | 39.0 | 39.1 | 38.8 | **39.9** | 38.7 | 39.1 | 38.8 | 38.9 | 38.8 | 38.6 | 38.7 | 39.0 |

| | | | | | | | | | | | | | | | | | |
|---|---|---|---|---|---|---|---|---|---|---|---|---|---|---|---|---|---|
| Numbers in bold indicate clinical fever. | | | | | | | | | | | | | | | | | |

All dogs were also monitored daily, from study day 33 through 48, for other clinical signs such as ocular discharge, nasal discharge, sneezing, coughing, dyspnea and depression. All dogs challenged with CIV and CIV plus *S.equi* (Groups 1, 3 and 4) exhibited range of clinical signs starting from two days post-challenge (Table 4 and Figure 1). Coughing and dyspnea were the predominant clinical signs. All dogs in Groups 1 (6/6) and 3 (10/10), exhibited varying degrees of coughing lasting for average of 2.7 days and 4.5 days, respectively. On the other hand, only 60% of the vaccinated dogs in Group 4 (6/10) exhibited a mild cough lasting for an average of 1.3 days. One dog (ID CAUELZ) in Group 1 was euthanized 9 days post-CIV challenge due to depression and dyspnea. One dog (ID CAUELW) in Group 3 was found dead 8 days after CIV challenge. None of the six dogs challenged with *S.equi* alone exhibited any clinical signs.

**Table 4: Clinical Scores following CIV challenge**

| **Group** | **Animal ID** | **Study Day** | | | | | | | | | | | | | | | | **Total Score** |
|---|---|---|---|---|---|---|---|---|---|---|---|---|---|---|---|---|---|---|
| | | 33 | 34 | **35*** | 36 | 37 | 38* * | 39 | 40 | 41 | 42 | 43 | 44 | 45 | 46 | 47 | 4 8 | |
| CIV Challenge (Group 1) | CAUELZ | 0 | 0 | 0 | 0 | 0 | 0 | 0 | 0.5^{C}, 2^{D} | 1^{C}, 2^{D} | 1^{C} | 2^{C}, 2^{D}, 2^{P}, 2^{F} | 2^{P}, 2^{D} | | | | | 18.5 |
| | CAUENF | 0 | 0 | 0 | 0 | 0 | 0 | 0 | 0 | 0 | 2^{D} | 1^{C}, 2^{D} | 0 | 0 | 0 | 0 | 0 | 5 |
| | CAUENR | 0 | 0 | 0 | 0 | 0 | 2^{S} | 0.5^{C} | 0 | 0.5^{C}, 2^{D}, 2^{F} | 0.5^{C}, 2^{D} | 1^{C}, 2^{D} | 1^{C} | 0 | 0 | 0 | 0 | 13.5 |
| | CAUEMP | 0 | 0 | 0 | 0 | 0 | 2^{S} | 0 | 2^{C} | 0.5^{C}, 2^{D} | 0 | 0 | 0 | 0 | 0 | 0 | 0 | 6.5 |
| | CAUELN | 0 | 0 | 0 | 0 | 0 | 0 | 2^{C} | 2^{C} | 0.5^{C}, 2^{D} | 0 | 0 | 1^{C} | 0 | 0 | 0 | 0 | 7.5 |
| | CAUEMZ | 0 | 0 | 0 | 0 | 0 | 0 | 0 | 0 | 0.5^{C} | 0 | 0 | 0 | 0 | 0 | 0 | 0 | 0.5 |
| *S*. *equi* Challenge (Group 2) | CAUELS | 0 | 0 | 0 | 0 | 0 | 0 | 0 | 0 | 0 | 0 | 0 | 0 | 0 | 0 | 0 | 0 | 0 |
| | CAUEME | 0 | 0 | 0 | 0 | 0 | 0 | 0 | 0 | 0 | 2^{F} | 0 | 0 | 0 | 0 | 0 | 0 | 2 |
| | CAUENM | 0 | 0 | 0 | 0 | 0 | 0 | 0 | 0 | 0 | 0 | 0 | 0 | 0 | 0 | 0 | 0 | 0 |
| | CAUENU | 0 | 0 | 0 | 0 | 0 | 0 | 0 | 0 | 0 | 0 | 0 | 0 | 0 | 0 | 0 | 0 | 0 |
| | CAUELM | 0 | 0 | 0 | 0 | 0 | 0 | 0 | 0 | 0 | 0 | 0 | 0 | 0 | 0 | 0 | 0 | 0 |
| | CAUELR | 0 | 0 | 0 | 0 | 0 | 0 | 0 | 0 | 0 | 0 | 0 | 0 | 0 | 0 | 0 | 0 | 0 |
| CIV plus S. *equi* Dual Challenge | CAUELU | 0 | 0 | 0 | 0 | 2^{F} | 0 | 2^{F} | 2^{C}, 2^{D}, 2^{F} | 1^{C}, 2^{D}, 2^{P}, 2^{F} | 2^{C}, 2^{D}, 1^{O} | 0.5^{C} | 0.5^{C} | 0.5 c | 0.5 c | 0 | 0 | 24 |
| | CAUELW | 0 | 0 | 0 | 0 | 2^{S} | 0 | 2^{F} | 2^{D} | 0.5^{C} 2^{D}, 2^{P}, 2^{F} | 2^{C}, 2^{D}, 1^{N}, 2^{P}, 2^{F} | | | | | | | 21.5 |
| | CAUENK | 0 | 0 | 0 | 0 | 2^{S} | 0 | 0 | 0 | 2^{D} | 0.5^{C}, 2^{D} | 2^{C}, 2^{D}, 2^{F} | 2^{S} | 2^{S} | 0 | 0 | 0 | 16.5 |
| | CAUELX | 0 | 0 | 0 | 0 | 0 | 0 | 1^{C} | 2^{C}, 2^{D}, 2^{F} | 2^{C} | 2^{C}, 2^{D}, 1^{N}, 2^{P}, 2^{F} | 2^{C} | 0 | 0 | 0.5 c | 0 | 0 | 20.5 |
| | CAUEPU | 0 | 0 | 0 | 0 | 0 | 0 | 2^{S} | 0 | 0.5^{C} | 0.5^{C} | 2^{C} | 0 | 2^{S} | 0 | 0 | 0 | 7 |
| | CAUENN | 0 | 0 | 0 | 0 | 0 | 0 | 0 | 2^{F} | 2^{P}, 1^{O}, 2^{F} | 2^{C} | 2^{C}, 2^{D} | 0.5^{C} | 0 | 0.5 c | 0 | 0 | 14 |
| | CAUEMR | 0 | 0 | 0 | 0 | 0 | 0 | 1^{O}, 2^{C} | 2^{S}, 1^{C}, 2^{D}, 2^{F} | 1^{O}, 2^{F} | 2^{C} | 2^{C}, 2^{D} | 0 | 0 | 0 | 0 | 0 | 19 |
| | CAUEMN | 0 | 0 | 0 | 2^{F} | 0 | 0.5^{C} | 2^{C} | 2^{S}, 0.5^{C}, 2^{F} | 2^{C} | 2^{C} | 0 | 0.5^{C} | 0 | 0 | 0 | 0 | 13.5 |
| | CAUELP | 0 | 0 | 0 | 0 | 0 | 2^{C} | 0.5^{C}, 2^{F} | 2^{C}, 2^{D}, 2^{F} | 0 | 0 | 2^{C}, 2^{D} | 0.5^{C} | 0 | 0.5 c | 0 | 0 | 15.5 |
| | CAUENA | 0 | 0 | 0 | 0 | 0 | 2^{S} | 0 | 2^{C}, 2^{D}, 2^{F} | 2^{P}, 1^{N}, 2^{C} | 2^{C}, 2^{D} | 2^{C} | 0.5^{C} | 0 | 0 | 0 | 0 | 19.5 |
| CIV Vaccinatio n and CIV plus *S. equi Dual* Challenge (Group 4) | CAUELT | 0 | 0 | 0 | 0 | 2^{F} | 0 | 0 | 0 | 0 | 0 | 0 | 0 | 0 | 0 | 0 | 0 | 2 |
| | CAUEN W | 0 | 0 | 0 | 0 | 0 | 2^{S} | 0 | 2^{C} | 2^{C} | 1^{C} | 2^{C} | 1^{C} | 0 | 0 | 0 | 0 | 10 |
| | CAUEPB | 0 | 0 | 0 | 0 | 0 | 0 | 0 | 0 | 0.5^{C} | 2^{S} | 0 | 0 | 0 | 0 | 0 | 0 | 2.5 |
| | CAUEMB | 0 | 0 | 0 | 0 | 2^{F} | 0 | 2^{C} | 0 | 0.5^{C} | 0 | 0 | 0 | 0 | 0 | 0 | 0 | 4.5 |
| | CAUENP | 0 | 0 | 0 | 0 | 0 | 0 | 2^{S} | 0 | 0 | 0 | 0 | 0 | 0 | 0 | 0 | 0 | 2 |
| | CAUEMS | 0 | 0 | 0 | 0 | 0 | 0 | 2^{C} | 0 | 0.5^{C}, 2^{D} | 0.5^{C}, 2^{F} | 0 | 0 | 0 | 0 | 0 | 0 | 7 |
| | CAUENL | 0 | 0 | 0 | 0 | 0 | 0 | 0 | 2^{F} | 0 | 0.5^{C} | 0 | 0 | 0 | 0 | 0 | 0 | 2.5 |
| | CAUEML | 0 | 0 | 0 | 0 | 0 | 0 | 0 | 0 | 0 | 0 | 0 | 0 | 0 | 0 | 0 | 0 | 0 |
| | CAUELL | 0 | 0 | 0 | 0 | 2^{S} | 0 | 0 | 0 | 0 | 0 | 0.5^{C} | 0 | 0 | 0 | 0 | 0 | 2.5 |
| | CAUEMX | 0 | 0 | 0 | 0 | 0 | 0 | 0 | 2^{F} | 0 | 0 | 0 | 0 | 0 | 0 | 0 | 0 | 2 |

| | | | | | | | | | | | | | | | | | | |
|---|---|---|---|---|---|---|---|---|---|---|---|---|---|---|---|---|---|---|
| O=Ocular Discharge; N=Nasal Discharge; C=Cough; S=Sneeze; D=Dyspnea; P=Depression; F=Fever;*CIV challenge; ** *S.equi* challenge | | | | | | | | | | | | | | | | | | |

The results suggest that CIV can cause severe respiratory disease in dogs that could lead to mortality. *S. equi* acts as secondary pathogen and enhances CIV-induced disease. *S. equi* alone did not induce any clinical disease on its own.

Virus Shedding: Nasal virus shedding was monitored in all CIV-challenged dogs (Groups 1, 3 and 4) on the day before challenge, and then, daily from day 1 through day 10 post-challenge. The average virus titer for each group, expressed as Log₁₀TCID₅₀/mL, was plotted against days post-challenge (Table 5 and Figure 2). Fifty percent of the dogs in Groups 1 and 4 and all dogs (100%) in Group 3 started shedding CIV in their nasal secretions from day 1 post-challenge. By 2 days post-challenge, all dogs in all three groups were positive for nasal virus shedding. The virus shedding reached its peak between 4 and 5 days CIV post-challenge followed by a precipitous drop on day 6 (Figure 2). Both non-vaccinates and vaccinates stopped shedding CIV in their nasal secretions by day 8 post-challenge. The virus titer for each dog was summed up over 10 days post-challenge period and statistical analysis was performed. The mean area under curve estimate was significantly higher for Groups 1 and 3 (17.0 Log₁₀ TCID₅₀/mL and 18.6 Log₁₀ TCID₅₀/mL, respectively) compared to Group 4 (8.9 Log₁₀ TCID₅₀/mL) (P=0.0075 and P<0.0001 for groups 1 and 3, respectively). The mean number of days of shedding in Groups 1 and 3 (5.7 days and 5.8 days, respectively) was also significantly higher (P=0.0356 and P=0.0033, respectively) compared to Group 4 (4 days). The results demonstrate that the CIV vaccine significantly reduced nasal virus shedding in vaccinated dogs.

**Table 5: Post-challenge nasal CIV shedding**

| **Treatment** | **Animal ID** | **Days Post-CIV Challenge** | | | | | | | | | | |
|---|---|---|---|---|---|---|---|---|---|---|---|---|
| | | **-1** | **1** | **2** | **3** | **4** | **5** | **6** | **7** | **8** | **9** | **10** |
| CIV Challenge (Group 1) | CAUELZ | 0 | 2.75 | 3.00 | 2.50 | 2.75 | 5.25 | 3.25 | 0 | 0 | 0 | |
| | CAUENF | 0 | 0.75 | 2.00 | 3.75 | 2.50 | 3.00 | 0.75 | 0 | 0 | 0 | 0 |
| | CAUENR | 0 | 0 | 4.00 | 3.75 | 6.25 | 5.00 | 3.00 | 0.75 | 0 | 0 | 0 |
| | CAUEMP | 0 | 2.50 | 5.00 | 3.75 | 2.75 | 4.00 | 1.75 | 2.00 | 0 | 0 | 0 |
| | CAUELN | 0 | 0 | 1.75 | 4.25 | 6.25 | 3.50 | 0 | 0 | 0 | 0 | 0 |
| | CAUEMZ | 0 | 0 | 2.00 | 0.75 | 3.00 | 2.25 | 1.75 | 0 | 0 | 0 | 0 |
| Average | | **0** | **1.00** | **2.96** | **3.13** | **3.92** | **3.83** | **1.75** | **0.46** | **0** | **0** | **0** |
| CIV plus *S.equi* Dual Challenge (Group 3) | CAUELU | 0 | 2.00 | 1.75 | 1.50 | 3.00 | 3.75 | 1.75 | 0 | 0 | 0 | 0 |
| | CAUELW | 0 | 3.50 | 3.00 | 2.75 | 2.75 | 5.50 | 4.25 | 0 | | | |
| | CAUENK | 0 | 0.75 | 3.75 | 3.50 | 3.75 | 1.00 | 1.50 | 0 | 0 | 0 | 0 |
| | CAUELX | 0 | 3.25 | 2.75 | 2.00 | 3.75 | 5.25 | 0 | 0 | 0 | 0 | 0 |
| | CAUEPU | 0 | 3.00 | 3.50 | 3.50 | 4.25 | 3.00 | 0 | 0 | 0 | 0 | 0 |
| | CAUENN | 0 | 2.75 | 2.00 | 2.00 | 4.75 | 5.50 | 2.25 | 0 | 0 | 0 | 0 |
| | CAUEMR | 0 | 3.00 | 3.75 | 3.00 | 3.00 | 6.00 | 3.50 | 1.50 | 0 | 0 | 0 |
| | CAUEMN | 0 | 1.50 | 2.75 | 2.75 | 3.25 | 5.25 | 4.75 | 0 | 0 | 0 | 0 |
| | CAUELP | 0 | 2.75 | 2.00 | 3.00 | 4.25 | 2.00 | 0 | 0 | 0 | 0 | 0 |
| | CAUENA | 0 | 3.25 | 2.25 | 4.00 | 4.75 | 5.50 | 4.50 | 0 | 0 | 0 | 0 |
| Average | | **0** | **2.58** | **2.75** | **2.80** | 3.75 | **4.28** | **2.25** | **0.15** | **0** | **0** | **0** |
| CIV Vaccination and CIV plus *S.equi* Dual Challenge (Group 4) | CAUELT | 0 | 0.75 | 3.25 | 0 | 0 | 1.25 | 1.50 | 0 | 0 | 0 | 0 |
| | CAUENW | 0 | 0 | 3.00 | 0 | 3.50 | 2.00 | 0.75 | 0 | 0 | 0 | 0 |
| | CAUEPB | 0 | 2.00 | 3.25 | 1.75 | 2.25 | 2.75 | 0 | 0 | 0 | 0 | 0 |
| | CAUEMB | 0 | 1.25 | 2.25 | 1.25 | 3.50 | 0 | 0 | 0 | 0 | 0 | 0 |
| | CAUENP | 0 | 0 | 1.00 | 0.75 | 0 | 0.75 | 0 | 0 | 0 | 0 | 0 |
| | CAUEMS | 0 | 0 | 2.50 | 2.25 | 1.75 | 3.75 | 0 | 0 | 0 | 0 | 0 |
| | CAUENL | 0 | 0 | 2.75 | 0 | 0 | 4.75 | 0 | 0 | 0 | 0 | 0 |
| | CAUEML | 0 | 1.25 | 5.00 | 2.75 | 1.50 | 2.50 | 0.75 | 0 | 0 | 0 | 0 |
| | CAUELL | 0 | 0 | 5.00 | 1.75 | 2.00 | 3.00 | 1.00 | 0.75 | 0 | 0 | 0 |
| | CAUEMX | 0 | 1.00 | 4.00 | 0 | 0 | 0 | 0 | 0 | 0 | 0 | 0 |
| Average | | **0** | **0.63** | **3.20** | **1.05** | **1.45** | **2.08** | **0.40** | **0.08** | **0** | **0** | **0** |

Bacterial Shedding: *S.equi* challenged dogs in Groups 2, 3 and 4 were monitored for nasal shedding of *S.equi* on days -4 (before CIV challenge), and at 0, 3, 6, 9 and 11 days after *S.equi* challenge (Table 6 and Figure 3). Additionally, lung lavage samples from all dogs in Groups 2, 3 and 4 were collected at the time of necropsy for *S.equi* isolation. All dogs in all groups, except those in Group 1 (not tested), were negative for *S.equi* shedding at the time of challenge with CIV or *S.equi.* None of the dogs challenged with *S.equi* alone (Group 2) was positive for nasal *S.equi* shedding at any time point after *S.equi* challenge. However, *S.equi* was isolated from the lung lavage sample of one dog (17%) in Group 2. In contrast, 100% of the dogs in Group 3, challenged with both CIV plus *S.equi,* were positive for *S.equi* in their nasal secretions 3 and 6 days after *S.equi* challenge and the majority of them (67%) continued to shed the bacteria for at least 9 days after challenge. The majority of the dogs in Group 3 (67%) were also positive for *S.equi* in their lung washes (11 days after *S.equi* challenge) suggesting that the bacteria colonized and multiplied in the lower respiratory tract. The majority (60%) of the CIV-vaccinated dogs challenged with CIV plus *S.equi* also shed *S.equi* in their nasal secretions 3 days after *S.equi* challenge. Six day onwards, the number of dogs shedding *S.equi* was substantially less in Group 4 compared to Group 3. Only 20% of the vaccinated dogs were positive for *S.equi* in their lung lavage samples.

**Table 6: Post-challenge S.equi isolation from nasal swabs and lung lavage**

| **Treatment** | **Days post-CIV challenge nasal *S.equi* isolation*** | | | | | | ***S.equi* isolation from lung wash** |
|---|---|---|---|---|---|---|---|
| | **-1** | **3** | **6** | **9** | **12** | **14** | |
| *S.equi* (Group 2) | 0/6 | 0/6 | 0/6 | 0/6 | 0/6 | 0/6 | 1/6 |
| CIV plus *S.equi* (Group 3) | 0/10 | 0/10 | 10/1 0 | 10/1 0 | 6/9 | 1/9 | 6/9 |
| CIV vaccination and CIV plus *S.equi* dual challenge (Group 4) | 0/10 | 0/10 | 6/10 | 3/10 | 4/10 | 1/10 | 2/10 |

| | | | | | | | |
|---|---|---|---|---|---|---|---|
| * Number of *S. equi*-positive dogs/Number of dogs in the group | | | | | | | |

Lung Lesions: Lung consolidation/pneumonia is the major pathological lesion in all influenza infections. All dogs (100%) in the CIV and the CIV plus *S.equi* challenge groups (Groups 1 and 3) exhibited varying degrees of lung consolidation, whereas only 3 dogs (30%) in the CIV vaccinated group (Group 4) developed lung consolidation of which two were very mild (Table 7). Only one dog (17%) in *S.equi* challenge group (Group 2) exhibited lung lesions and they were mild. In Group 1, the lung lesions were predominantly observed in cranial lobes whereas in Group 3, the lesions appeared to be present in all lung lobes. Lung lesions were characterized by hemorrhages and reddish consolidation and hepatization. The lung scores in Group 1 ranged between 0.5 and 32.97 with a median score=17.27, and in Group 3, between 5.53 and 39.74 with a median lung score=23.29. In Group 4, the scores ranged between 0 and 25.54 with median score=0. Mitigated fraction estimate of vaccine efficacy relative to CIV challenge was 88.3% at 95% CI (60%, 100%), and relative to CIV plus *S.equi* challenge was 88.0% at 95% CI (58%, 100%). The lung scores in Group 4 were significantly lower compared to Group 1 (P=0.0019) and Group 3 (P=0.0002). Although the lung scores in Group 3 were higher compared to Group 1, they were not significantly different (P=0.5622). The data demonstrates that the CIV vaccine aids in the prevention of CIV-and CIV plus *S.equi-*induced lung lesions.

**Table 7: Lung lesion scores following CIV and/or S.equi challenge**

| **Treatment** | **Animal ID** | **Raw Scores** | | | | | | | **Weighted Scores** | | | | | | | **Total Score** |
|---|---|---|---|---|---|---|---|---|---|---|---|---|---|---|---|---|
| | | **R. Cranial** | **R. Middle** | **R. Caudal** | **R. Access** | **L.Cr-Cr** | **L. Cr-Cau** | L. **Caudal** | **R. Cranial** | **R. Middle** | **R. Caudal** | **R. Access** | **L. Cr-Cr** | **L.Cr-Cau** | **L. Caudal** | |
| **CIV Challenge (Group 1)** | CAUELZ | 0 | 0 | 0 | 0 | 50 | 75 | 90 | 0 | 0 | 0 | 0 | 4.55 | 4.50 | 23.31 | 32.36 |
| | CAUENF | 0 | 0 | 0 | 0 | 10 | 0 | 0 | 0 | 0 | 0 | 0 | 0.91 | 0 | 0 | 0.91 |
| | CAUENR | 5 | 100 | 5 | 70 | 90 | 70 | 5 | 0.76 | 10.00 | 1.24 | 6.30 | 8.19 | 4.20 | 1.30 | 31.99 |
| | CAUEMP | 0 | 5 | 0 | 0 | 0 | 0 | 0 | 0 | 0.50 | 0 | 0 | 0 | 0 | 0 | 0.50 |
| | CAUELN | 0 | 5 | 0 | 0 | 5 | 5 | 5 | 0 | 0.50 | 0 | 0 | 0.46 | 0.30 | 1.30 | 2.55 |
| | CAUEMZ | 90 | 30 | 0 | 90 | 90 | 0 | 0 | 13.68 | 3.00 | 0 | 8.10 | 8.19 | 0 | 0 | 32.97 |
| | | **Mean** | | | | | | | | | | | | | | **16.88** |
| **S.equi Challenge (Group 2)** | CAUELS | 0 | 0 | 0 | 0 | 0 | 0 | 0 | 0 | 0 | 0 | 0 | 0 | 0 | 0 | 0 |
| | CAUEME | 0 | 0 | 0 | 0 | 0 | 0 | 0 | 0 | 0 | 0 | 0 | 0 | 0 | 0 | 0 |
| | CAUENM | 0 | 0 | 0 | 0 | 5 | 10 | 2 | 0 | 0 | 0 | 0 | 0.46 | 0.60 | 0.52 | 1.57 |
| | CAUENU | 0 | 0 | 0 | 0 | 0 | 0 | 0 | 0 | 0 | 0 | 0 | 0 | 0 | 0 | 0 |
| | CAUELM | 0 | 0 | 0 | 0 | 0 | 0 | 0 | 0 | 0 | 0 | 0 | 0 | 0 | 0 | 0 |
| | CAUELR | 0 | 0 | 0 | 0 | 0 | 0 | 0 | 0 | 0 | 0 | 0 | 0 | 0 | 0 | 0 |
| | | **Mean** | | | | | | | | | | | | | | **0.26** |
| **CIV plus** *S.equi* **Dual Challenge (Group 3)** | CAUELU | 10 | 70 | 10 | 60 | 30 | 0 | 10 | 1.52 | 7.00 | 2.48 | 5.40 | 2.73 | 0 | 2.59 | 21.72 |
| | CAUELW | 85 | 10 | 40 | 10 | 10 | 10 | 10 | 12.92 | 1.00 | 9.92 | 0.90 | 0.91 | 0.60 | 2.59 | 28.84 |
| | CAUENK | 30 | 70 | 1 | 0 | 0 | 0 | 0 | 4.56 | 7.00 | 0.25 | 0 | 0 | 0 | 0 | 11.81 |
| | CAUELX | 2 | 90 | 20 | 50 | 10 | 0 | 20 | 0.30 | 9.00 | 4.96 | 4.50 | 0.91 | 0.00 | 5.18 | 24.85 |
| | CAUEPU | 10 | 30 | 0 | 5 | 2 | 2 | 1 | 1.52 | 3.00 | 0 | 0.45 | 0.18 | 0.12 | 0.26 | 5.53 |
| | CAUENN | 40 | 100 | 15 | 70 | 60 | 50 | 20 | 6.08 | 10.00 | 3.72 | 6.30 | 5.46 | 3.00 | 5.18 | 39.74 |
| | CAUEMR | 10 | 95 | 15 | 70 | 70 | 50 | 15 | 1.52 | 9.50 | 3.72 | 6.30 | 6.37 | 3.00 | 3.89 | 34.30 |
| | CAUEMN | 0 | 80 | 10 | 5 | 60 | 0 | 5 | 0 | 8.00 | 2.48 | 0.45 | 5.46 | 0 | 1.30 | 17.69 |
| | CAUELP | 0 | 95 | 10 | 10 | 40 | 0 | 0 | 0 | 9.50 | 2.48 | 0.90 | 3.64 | 0 | 0 | 16.52 |
| | CAUENA | 10 | 40 | 10 | 40 | 80 | 50 | 25 | 1.52 | 4.00 | 2.48 | 3.60 | 7.28 | 3.00 | 6.48 | 28.36 |
| | | **Mean** | | | | | | | | | | | | | | **22.93** |
| **CIV Vaccination and CIV plus** *S.equi* **Dual Challenge) (Group 4)** | CAUELT | 0 | 5 | 0 | 0 | 0 | 0 | 0 | 0 | 0.50 | 0 | 0 | 0 | 0 | 0 | 0.50 |
| | CAUENW | 0 | 0 | 0 | 0 | 0 | 0 | 0 | 0 | 0 | 0 | 0 | 0 | 0 | 0 | 0 |
| | CAUEPB | 0 | 0 | 0 | 0 | 0 | 0 | 0 | 0 | 0 | 0 | 0 | 0 | 0 | 0 | 0 |
| | CAUEMB | 0 | 0 | 0 | 0 | 0 | 0 | 0 | 0 | 0 | 0 | 0 | 0 | 0 | 0 | 0 |
| | CAUENP | 0 | 0 | 0 | 0 | 0 | 0 | 0 | 0 | 0 | 0 | 0 | 0 | 0 | 0 | 0 |
| | CAUEMS | 10 | 70 | 10 | 30 | 60 | 20 | 20 | 1.52 | 7.00 | 2.48 | 2.70 | 5.46 | 1.20 | 5.18 | 25.54 |
| | CAUENL | 0 | 0 | 0 | 0 | 0 | 0 | 0 | 0 | 0 | 0 | 0 | 0 | 0 | 0 | 0 |
| | CAUEML | 0 | 0 | 0 | 0 | 0 | 0 | 0 | 0 | 0 | 0 | 0 | 0 | 0 | 0 | 0 |
| | CAUELL | 0 | 0 | 0 | 0 | 0 | 0 | 1 | 0 | 0 | 0 | 0 | 0 | 0 | 0.26 | 0.26 |
| | CAUEMX | 0 | 0 | 0 | 0 | 0 | 0 | 0 | 0 | 0 | 0 | 0 | 0 | 0 | 0 | 0 |
| | | **Mean** | | | | | | | | | | | | | | **2.63** |

## Claims

1. A vaccine consisting of an inactivated canine influenza virus and optionally an adjuvant for use in the protection of a canine against *Streptococcus equi.*

2. A combination vaccine consisting of an inactivated canine influenza virus and one or more additional immunogens and optionally an adjuvant, for use in the protection of a canine against *Streptococcus equi*,
wherein the one or more additional immunogens is selected from the group consisting of canine distemper virus, canine adenovirus, canine parvovirus, canine parainfluenza virus, canine coronavirus, canine herpesvirus, canine pneumovirus, Leptospira serovars, Leishmania organisms, a Borrelia species (spp.); Bordetella bronchiseptica, Mycoplasma species, rabies virus, Ehrlichia canis.

3. A vaccine for use according to claim 1 or 2 wherein *Streptococcus equi is Streptococcus equi* subsp. *Zooepidemicus.*

4. A vaccine for use according to anyone of claim 1 to 3 wherein the inactivated canine influenza virus is a killed canine influenza virus.

## Patentansprüche

1. Impfstoff, bestehend aus einem inaktivierten Hundeinfluenzavirus und gegebenenfalls einem Adjuvans zur Verwendung im Schutz eines Hundes gegen *Streptococcus equi.*

2. Kombinationsimpfstoff, bestehend aus einem inaktivierten Hundeinfluenzavirus und einem oder mehreren zusätzlichen Immunogenen und gegebenenfalls einem Adjuvans zur Verwendung im Schutz eines Hundes gegen *Streptococcus equi,*
wobei das eine oder die mehreren zusätzlichen Immunogene aus der Gruppe bestehend aus Hundestaupevirus, Hundeadenovirus, Hundeparvovirus, Hundeparainfluenzavirus, Hundecoronavirus, Hundeherpesvirus, Hundepneumovirus, Leptospira-Serovaren, Leishmania-Organismen, einer Borrelia-Spezies (ssp.); Bordetella bronchiseptica, Mycoplasma-Spezies, Tollwutvirus, Ehrlichia canis ausgewählt sind.

3. Impfstoff zur Verwendung nach Anspruch 1 oder 2, wobei es sich bei *Streptococcus equi* um *Streptococcus equi* subsp. *Zooepidemicus* handelt.

4. Impfstoff zur Verwendung nach einem der Ansprüche 1 bis 3, wobei es sich bei dem inaktivierten Hundeinfluenzavirus um ein abgetötetes Hundeinfluenzavirus handelt.

## Revendications

1. Vaccin constitué d'un virus influenza canin inactivé et éventuellement d'un adjuvant pour utilisation dans la protection d'un canin contre *Streptococcus equi.*

2. Vaccin combiné constitué d'un virus influenza canin inactivé et d'un ou de plusieurs immunogènes supplémentaires, et éventuellement d'un adjuvant, pour utilisation dans la protection d'un canin contre *Streptococcus equi,*
où le ou les immunogènes supplémentaires sont choisis dans le groupe constitué par : virus de la maladie de Carré, adénovirus canin, parvovirus canin, virus parainfluenza canin, coronavirus canin, herpèsvirus canin, pneumovirus canin, *Leptospira serovars,* organismes du genre *Leishmania,* espèce de *Borrelia* (*spp.*) ; *Bordetella bronchiseptica,* espèce de *Mycoplasma,* virus de la rage, *Ehrlichia canis.*

3. Vaccin pour utilisation selon la revendication 1 ou 2, où *Streptococcus equi* est *Streptococcus equi* de la sous-espèce *Zooepidemicus.*

4. Vaccin pour utilisation selon l'une quelconque des revendications 1 à 3, où le virus influenza canin inactivé est un virus influenza canin détruit.
